# EUROPEAN PATENT APPLICATION

(11) **EP 4 249 062 A1**
(43) Date of publication of application: **27.09.2023**
(21) Application number: 22164161.6
(22) Date of filing: 24.03.2022
(51) Int. Cl.: A61P 35/00, C07K 14/705, C07K 14/74, A61P 25/00

(54) **MHC IB-MEDIATED AQUAPORIN 4 (AQP4)-SPECIFIC IMMUNOSUPPRESSION AS A NOVEL TREATMENT FOR NMO**

(71) Applicant: Julius-Maximilians-Universität Würzburg, 97070 Würzburg (DE)
(72) Inventor: BRUTTEL, Valentin., 97080 Würzburg (DE); WISCHHUSEN, Jörg., 97082 Würzburg (DE); JAYARAM, Shriya., 97078 Würzburg (DE); AHSAN, Fadhil., 10555 Berlin (DE)
(74) Representative: Hoffmann Eitle

(57) **Abstract**

The present invention relates to therapeutical uses of non-classical human major histocompatibility complex (MHC) molecules (also named MHC class lb molecules) in combination with peptide antigens for the treatment of neuromyelitis optica (NMO). The invention more specifically relates to recombinant polypeptides comprising peptide antigens and one or more domains of a non-classical MHC class lb molecule. The invention also relates to methods of producing such recombinant polypeptides, pharmaceutical compositions comprising the same, as well as their uses for treating neuromyelitis optica (NMO).

## Description

### FIELD OF THE INVENTION

The present invention relates to therapeutical uses of non-classical human major histocompatibility complex (MHC) molecules (also named MHC class Ib molecules) in combination with peptide antigens for the treatment of neuromyelitis optica (NMO). The invention more specifically relates to recombinant polypeptides comprising peptide antigens and one or more domains of a non-classical MHC class Ib molecule. The invention also relates to methods of producing such recombinant polypeptides, pharmaceutical compositions comprising the same, as well as their uses for treating neuromyelitis optica (NMO).

### BACKGROUND

Neuromyelitis optica (NMO), a demyelinating autoimmune disease of the central nervous system (CNS) with an incidence between 0.05 and 0.4/100,000. It is characterized by similar symptoms as Multiple Sclerosis. However, whereas Multiple Sclerosis is often characterized by a relapsing-remitting couse of disease, patients with NMO rarely experience remissions. Moreover, some treatments that are beneficial in patients with MS fail in NMO or may even aggravate the disease. Therefore, NMO is more difficult to treat than MS and usually progesses faster.Current therapeutic strategies for NMO focus on relapse prevention or treatment of acute disease relapses. Acute relapses are usually treated with immunosuppressive therapeutics such as glucocorticoids or plasmapheresis to remove autoreactive antibodies. More recently, therapeutic antibodies that inhibit the complement system (eculizumab) or block inflammatory cytokines (satralizumab) have also been approved.

However, not only autoreactive but also protective immune functions are inhibited. These are essential to protect the patient from viruses, bacteria or tumors. Accordingly, often only partial inhibition can be targeted. Since autoimmune diseases usually worsen after recurrent attacks, a great deal of effort is put into relapse prevention. However, a general slow progression of this autoimmune disease can only be slowed but not prevented. To circumvent this dilemma, attempts have been made for some time to induce immunological tolerance to antigens attacked by autoreactive immune cells (antigen-specific immunosuppression, ASI).

Two strategies have been evaluated so far, at least in early clinical studies. Similar to hyposensitization strategies in allergy, large amounts of antigens have been administered by different routes to induce antigen-specific tolerance. However, in autoimmune diseases, these strategies have caused severe side effects and have not been clinically successful. Attempts to induce tolerance by adoptive transfer of antigen-specific regulatory T cells or antigen-loaded tolerogenic dendritic cells appear more promising. However, these strategies are extremely complex and expensive, requiring GMP-compliant production and quality control processes for each individual patient. Therefore, even if small clinical trials have been successful, it is highly questionable whether adoptive transfer therapies will be available for many patients in the foreseeable future. WO 2018/215340 relates to combinations of MHC class Ib molecules and peptides for targeted therapeutic immunomodulation.

Taken together, there remains a need for improved drugs for the treatment of neuromyelitis optica (NMO).

### DESCRIPTION OF THE INVENTION

The inventors have found that human MHC class Ib molecules such as HLA-G possess the ability to induce antigen-specific tolerance towards presented peptide antigens. Thus, albeit being of similar structure and sequence as classical human MHC class la molecules which induce antigen peptide-specific immune responses, MHC class Ib molecules can advantageously be used according to the invention to suppress immune responses in an antigen-specific manner. Additionally, the inventors have found that for the suppression of immune responses according to the invention, molecules other than naturally occurring MHC class Ib molecules, and in particular polypeptides which only comprise at least one domain of an MHC class Ib molecule, preferably at least an [alpha]3 domain of an MHC class Ib molecule, can be used: The [alpha]1 and [alpha]2 domains of variable class I a molecules can be combined with the [alpha]3 domain of a human MHC class Ib molecule in order to suppress immune responses towards peptides presented by these antigens.

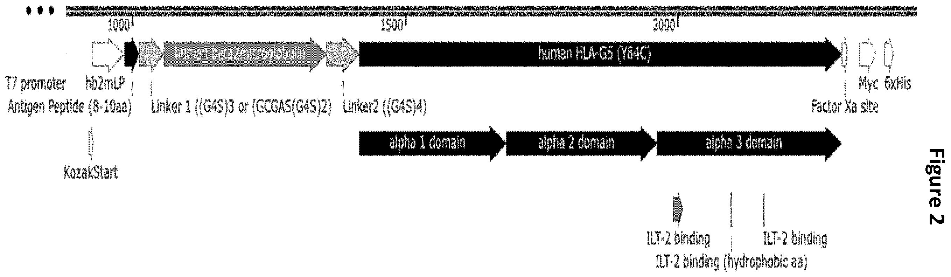

Antigen-loaded HLA-G molecules can be unstable. Thus, the inventors designed soluble recombinant polypeptides comprising a peptide antigen, an MHC class Ib molecule such as HLA-G and β2-microglobulin (b2m), and connected these three components covalently (e.g., via covalent linkers). Alternatively, the antigen-binding α1 and α2 domains of an MHC class Ib molecule such as HLA-G were exchanged by the respective domains of other MHC molecules to enhance the flexibility and versatility of these recombinant polypeptides (see, for instance, Figure 2). These alternative recombinant polypeptides can be designed with antigen-binding domains of other human HLA molecules. It was previously found that constructs comprising the α1 and α2 domains of murine H2-K^{b} can present the ovalbumin-derived peptide SIINFEKL to OT-1 T cells. (OT-1 T cells express a transgenic T cell receptor that specifically recognizes this antigen) (WO 2018/215340).

Surprisingly, the inventors have found that by using the recombinant polypeptides of the invention, immune responses against neuroinflammatory autoantigens can be suppressed and cells that induce tolerance to human aquaporin 4 (AQP4) can be induced. Further, using established models for neuroinflammatory conditions, the inventors have shown that surrogates of these polypeptides (adapted for use in mice) can be used to treat neuroinflammatory diseases. Thus, according to the invention, neuromyelitis optica (NMO) can be treated by the recombinant polypeptides of the invention.

Experimental data of the inventors show that a suitable peptide antigen and the presence of an [alpha]3 domain of an MHC class Ib molecule (e.g. HLA-G) are required to achieve the desired effect. Therefore, this approach goes beyond previously described strategies that either use antigenic peptides in the absence of costimulation (resulting in anergic rather than tolerogenic T cells), or MHC class Ib molecules in an antigen-unspecific setting.

Moreover, according to the invention, the recombinant polypeptides of the invention do not only modulate T-cell responses but also prevent the formation of aquaporin 4 (AQP4)-specific autoantibodies. It is expected that this advantage will translate into a clinical improvement in human patients having neuromyelitis optica (NMO), because aquaporin 4 (AQP4)-specific autoantibodies are involved in the pathology of neuromyelitis optica (NMO).

Accordingly, the invention relates to the following preferred embodiments:
1. A recombinant polypeptide capable of presenting a peptide antigen, the recombinant polypeptide comprising, in an N- to C-terminal order,
   i) a peptide antigen presented by said recombinant polypeptide, wherein the peptide antigen is a peptide of human aquaporin 4;
   ii) optionally a linker sequence;
   iii) optionally a sequence of a human polypeptide domain comprising a sequence of a human β2 microglobulin, or an amino acid sequence at least 90% identical to the amino acid sequence of human β2 microglobulin represented by SEQ ID NO: 5;
   iv) optionally a linker sequence;
   v) optionally an [alpha] 1 domain of an MHC molecule;
   vi) optionally an [alpha] 2 domain of an MHC molecule;
   vii) an [alpha] 3 domain of an MHC class Ib molecule or a derivative of an [alpha] 3 domain of an MHC class Ib molecule, said derivative being capable of binding to ILT2 or ILT4;
   viii) optionally a protease cleavage site;
   ix) optionally a spacer sequence; and
   x) optionally an affinity tag.
2. The recombinant polypeptide according to item 1, wherein said peptide antigen according to i) is 7 to 11 amino acids in length, preferably 8-10 amino acids in length.
3. The recombinant polypeptide according to item 1 or 2, wherein said peptide antigen according to i) consists of an amino acid sequence selected from the group consisting of the amino acid sequences of SEQ ID NOs: 2, 22, 23 and 24.
4. The recombinant polypeptide according to any one of the preceding items, wherein said peptide antigen consists of the amino acid sequence of SEQ ID NO: 2 or SEQ ID NO: 22.
5. The recombinant polypeptide according to any one of items 1-4, wherein said peptide antigen consists of the amino acid sequence of SEQ ID NO: 2.
6. The recombinant polypeptide according to any one of items 1-3, wherein said peptide antigen consists of the amino acid sequence of SEQ ID NO: 23 or SEQ ID NO: 24.
7. The recombinant polypeptide according to any one of the preceding items, wherein said [alpha]1 domain according to (v) and said [alpha]2 domain according to (vi) are from a human MHC class Ia molecule or from a human MHC class Ib molecule.
8. The recombinant polypeptide according to any one of the preceding items, wherein said [alpha]1 domain according to (v) and said [alpha]2 domain according to (vi) are from a human MHC class la molecule.
9. The recombinant polypeptide according to item 8, wherein said [alpha]1 domain according to (v) and said [alpha]2 domain according to (vi) are from a human HLA-A2 molecule.
10. The recombinant polypeptide according to item 7, wherein said [alpha]1 domain according to (v) and said [alpha]2 domain according to (vi) are from a human MHC class Ib molecule.
11. The recombinant polypeptide according to item 10, wherein said [alpha]1 domain according to (v) and said [alpha]2 domain according to (vi) are from a human HLA-G molecule.
12. The recombinant polypeptide according to any one of the preceding items, wherein the [alpha] 3 domain of the MHC class Ib molecule according to (vii) is an [alpha] 3 domain of human HLA-E, human HLA-F or human HLA-G.
13. The recombinant polypeptide according to any one of the preceding items, wherein the [alpha] 3 domain of the MHC class Ib molecule according to (vii) is an [alpha] 3 domain of human HLA-G.
14. The recombinant polypeptide according to any one of the preceding items, wherein the [alpha]3 domain or derivative according to (vii) is identical to or has at least 80% amino acid sequence identity, preferably at least 90% amino acid sequence identity, with the [alpha]3 domain having the amino acid sequence of SEQ ID NO: 9 or SEQ ID NO: 21.
15. The recombinant polypeptide according to item 14, wherein the [alpha]3 domain or derivative according to (vii) is identical to or has at least 92% amino acid sequence identity with the [alpha]3 domain having the amino acid sequence of SEQ ID NO: 9 or SEQ ID NO: 21.
16. The recombinant polypeptide according to item 14, wherein the [alpha]3 domain or derivative according to (vii) is identical to or has at least 94% amino acid sequence identity with the [alpha]3 domain having the amino acid sequence of SEQ ID NO: 9 or SEQ ID NO: 21.
17. The recombinant polypeptide according to item 14, wherein the [alpha]3 domain or derivative according to (vii) is identical to or has at least 96% amino acid sequence identity with the [alpha]3 domain having the amino acid sequence of SEQ ID NO: 9 or SEQ ID NO: 21.
18. The recombinant polypeptide according to item 14, wherein the [alpha]3 domain or derivative according to (vii) is identical to or has at least 98% amino acid sequence identity with the [alpha]3 domain having the amino acid sequence of SEQ ID NO: 9 or SEQ ID NO: 21.
19. The recombinant polypeptide according to item 14, wherein the [alpha]3 domain or derivative according to (vii) is identical to or has at least 99% amino acid sequence identity with the [alpha]3 domain having the amino acid sequence of SEQ ID NO: 9 or SEQ ID NO: 21.
20. The recombinant polypeptide according to item 14, wherein the [alpha]3 domain according to (vii) is identical to the [alpha]3 domain having the amino acid sequence of SEQ ID NO: 9 or SEQ ID NO: 21.
21. The recombinant polypeptide according to any one of the preceding items, wherein the linker sequence according to (ii) and/or the linker sequence according to (iv) comprises the amino acid sequence (GGGGS)n, wherein n is an integer equal to or higher than 1.
22. The recombinant polypeptide according to item 21, wherein the linker sequence according to (ii) comprises the amino acid sequence (GGGGS)n, and wherein n is an integer selected from the group consisting of 1, 2, 3, 4, 5, 6, 7, 8, 9 and 10 and is preferably selected from the group consisting of 2, 3, 4 and 5.
23. The recombinant polypeptide according to item 21 or 22, wherein the linker sequence according to (iv) comprises the amino acid sequence (GGGGS)n, and wherein n is an integer selected from the group consisting of 1, 2, 3, 4, 5, 6, 7, 8, 9 and 10 and is preferably selected from the group consisting of 2, 3, 4 and 5.
24. The recombinant polypeptide according to any one of the preceding items, wherein said sequence of a human polypeptide domain according to (iii) is at least 95% identical to the amino acid sequence of SEQ ID NO: 5, preferably at least 98% identical to the amino acid sequence of SEQ ID NO: 5 and more preferably identical to the amino acid sequence of SEQ ID NO: 5.
25. The recombinant polypeptide according to any one of the preceding items, wherein said polypeptide is dimeric or multimeric.
26. The recombinant polypeptide according to any one of the preceding items, wherein the polypeptide comprises or consists of all of the components i) to vii)
27. The recombinant polypeptide according to any one of the preceding items, wherein the polypeptide does not comprise components viii) to x).
28. The recombinant polypeptide according to any one of items 1 to 26, wherein the polypeptide comprises or consists of all of the components i) to x).
29. The recombinant polypeptide according to any one of the preceding items, further comprising an N-terminal secretion signal peptide sequence.
30. The recombinant polypeptide according to any one of items 1-28, wherein the recombinant polypeptide consists of an amino acid sequence consisting of the following ((a) and (b)) in an N-to C-terminal order:
   (a) a peptide antigen selected from the group consisting of the amino acid sequences of SEQ ID NOs: 2, 22, 23 and 24, and
   (b) the amino acid sequence of SEQ ID NO: 16.
31. The recombinant polypeptide according to any one of the preceding items, wherein the recombinant polypeptide is soluble.
32. A nucleic acid encoding one or more polypeptides according to any one of the preceding items.
33. The nucleic acid according to item 32, wherein the nucleic acid is a vector.
34. A pharmaceutical composition or kit comprising at least one nucleic acid according to items 32 or 33.
35. A pharmaceutical composition or kit comprising at least one recombinant polypeptide according to any one of items 1-31.
36. The pharmaceutical composition or kit according to item 35, wherein the pharmaceutical composition or kit comprises at least two different recombinant polypeptides according to any one of items 1-31, and wherein each of the different polypeptides comprises a different peptide antigen as defined in any one of items 3 to 6.
37. A pharmaceutical composition or kit according to any one of items 34-36, for use in the treatment of neuromyelitis optica in a human patient.
38. The pharmaceutical composition or kit for use according to item 37, wherein the treatment is treatment by immunotherapy.
39. The pharmaceutical composition or kit for use according to any one of items 37-38, wherein the treatment is by inducing immunological tolerance against human aquaporin 4.
40. The pharmaceutical composition or kit for use according to any one of items 37-39, wherein the treatment is for reducing plasma or cerebrospinal fluid levels of autoantibodies against human aquaporin 4.
41. The pharmaceutical composition or kit for use according to any one of items 37-40, wherein the human patient is a patient who had plasma or cerebrospinal fluid autoantibodies against human aquaporin 4 prior to the start of the treatment.
42. The pharmaceutical composition or kit for use according to any one of items 37-41, wherein the treatment is by inducing myelin-specific regulatory T cells.
43. A recombinant host cell comprising a nucleic acid or a vector according to item 32 or 33 and expressing the recombinant polypeptide according to any one of items 1-31.
44. A method for obtaining pharmaceutical composition comprising a polypeptide according to any one of items 1-31, the method comprising the steps of (a) culturing the recombinant host cell of item 43 under conditions allowing expression of the recombinant polypeptide from the nucleic acid molecule, (b) recovering the recombinant polypeptide, (c) purifying the recombinant polypeptide, and (d) formulating the recombinant polypeptide into a pharmaceutical composition.

### BRIEF DESCRIPTION OF THE DRAWINGS

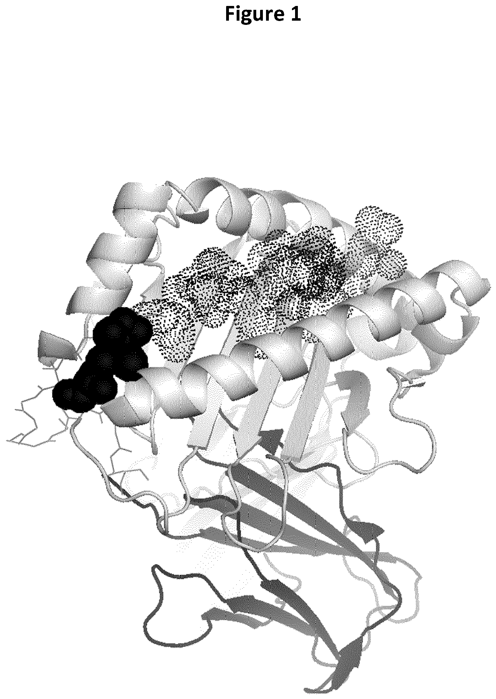

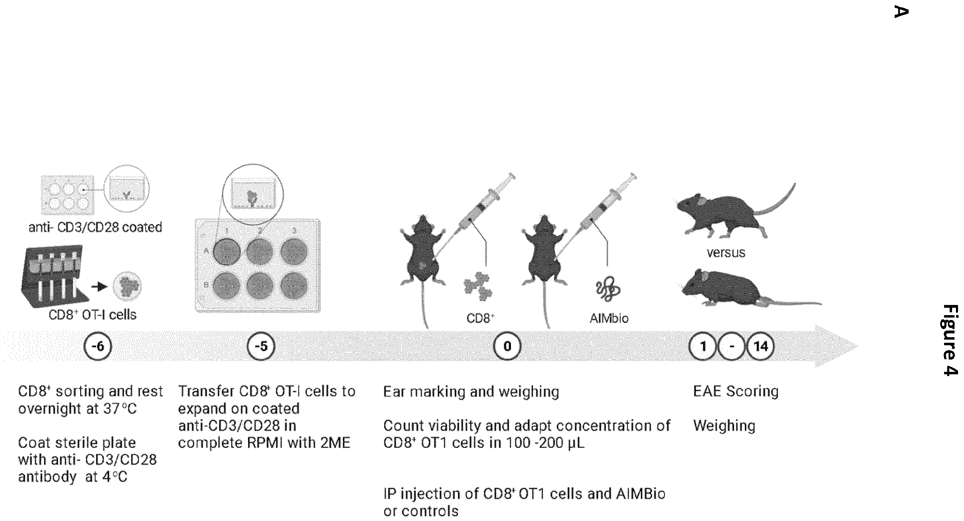

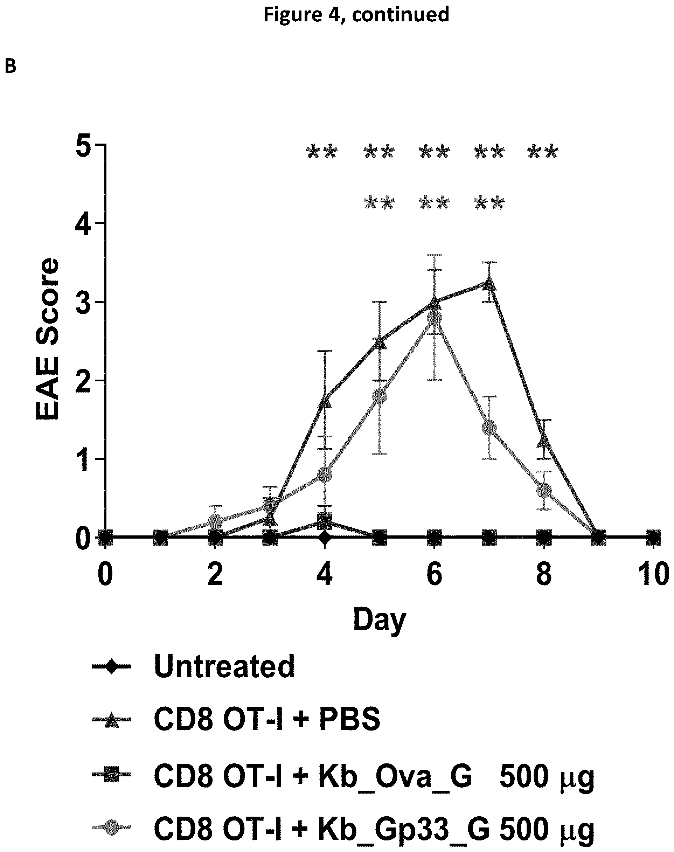

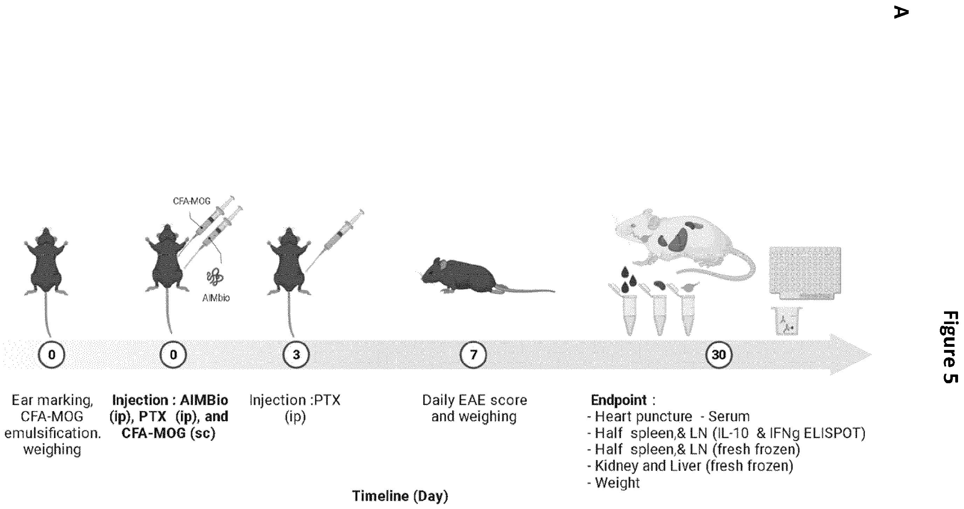

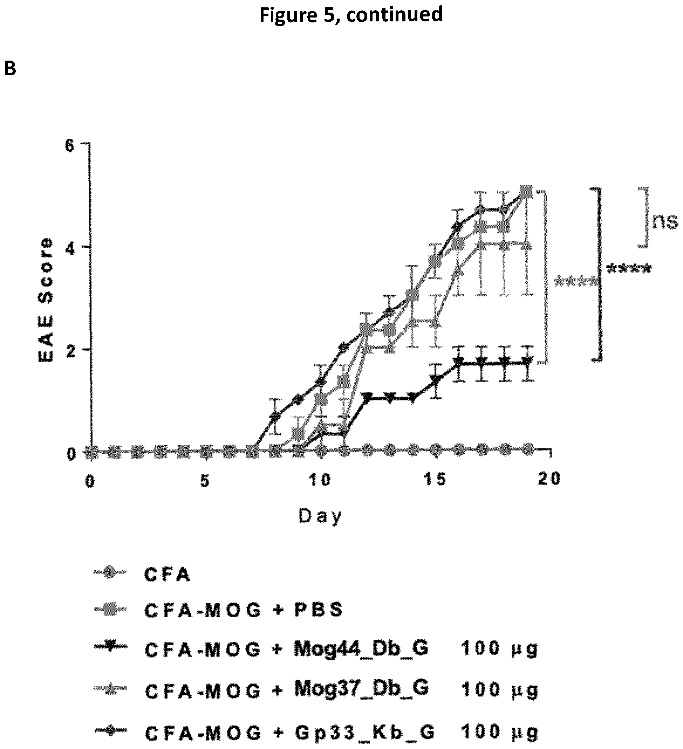

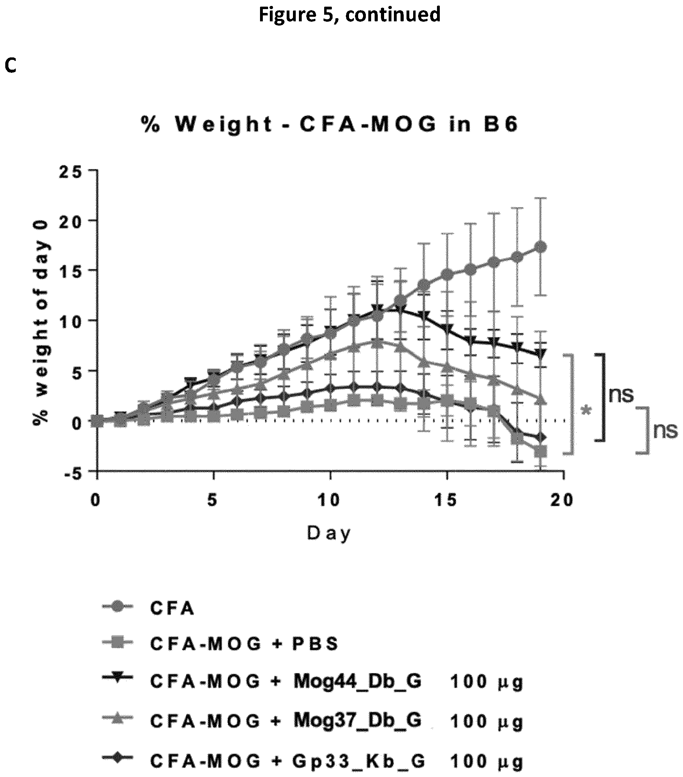

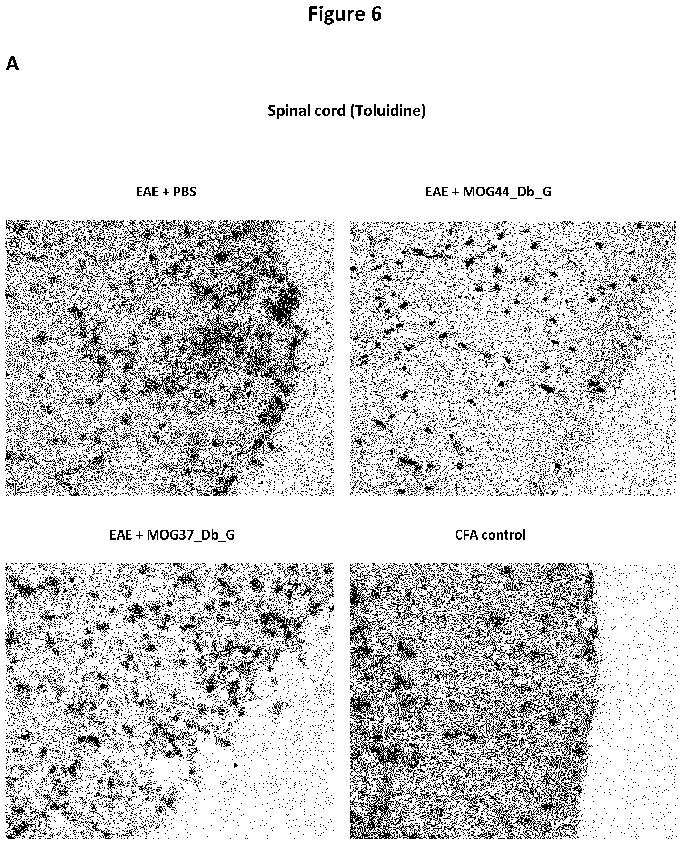

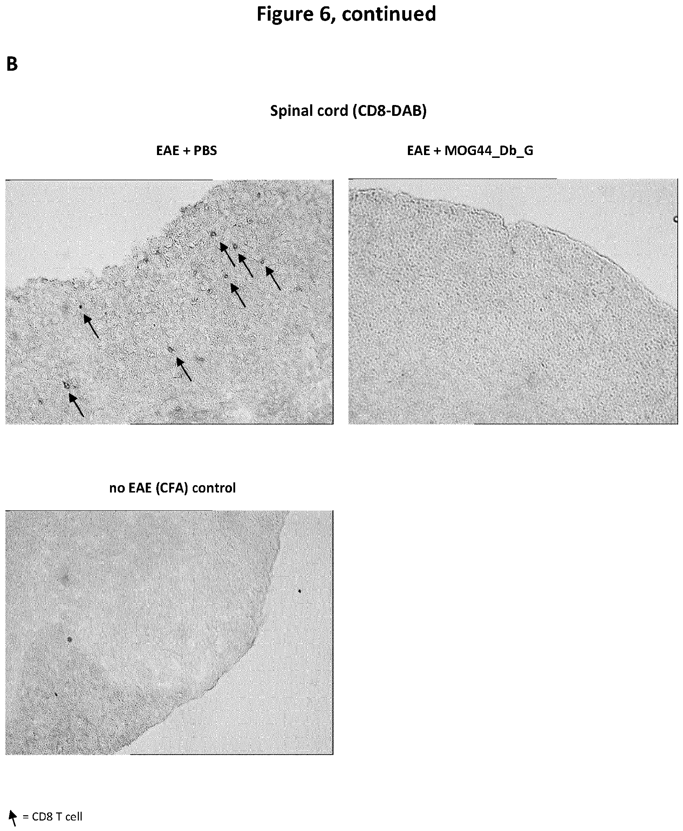

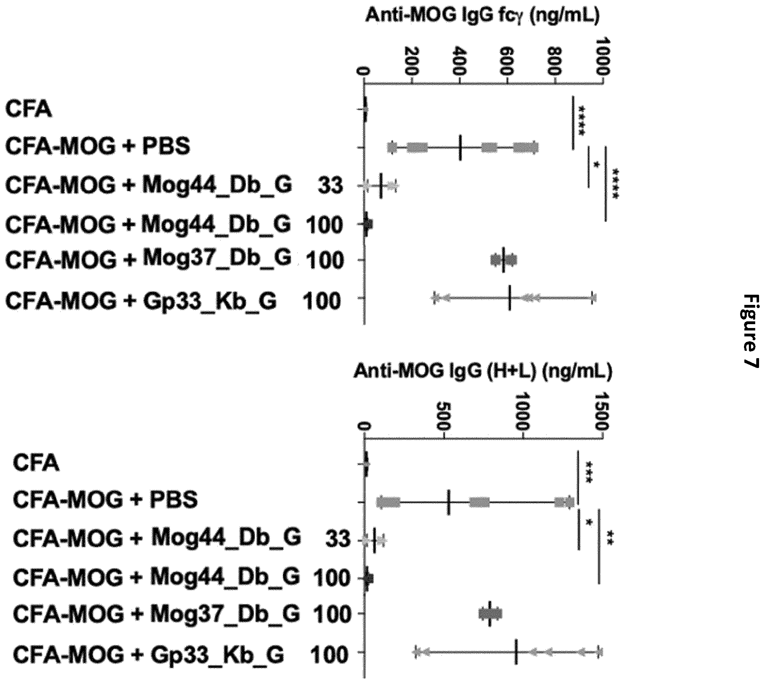

Figure 1: Depiction of a peptide-loaded soluble MHC Ib molecule suitable to achieve therapeutic antigen-specific immunomodulation.
The presented peptide antigenis depicted in dotted spheres, the HLA-G alpha1-3 domains are sketched in light-grey, and the beta2microglobulin domain is shown in dark grey. An optional linker connecting the antigenic peptide with the beta2microglobulin molecule is displayed in grey stick style, and an optional disulfide trap is depicted in black spheres. This figure was generated using Pymol and is adapted from structures published in Clements et al., Proc Natl Acad Sci U S A. 2005 Mar 1;102(9):3360-5 and Hansen et al., Trends Immunol. 2010 Oct;31(10):363-9.
Figure 2: Example for a vector-based construct encoding a single chain MHC Ib molecule suitable for therapeutic peptide-specific immunomodulation.
HLA-G1 and HLA-G5 each consist of 3 [alpha] domains (here in black), a non-covalently associated beta 2-microglobulin subunit (here in dark grey) and the antigenic peptide presented on HLA-G (short black arrow). HLA-G1 further contains a transmembrane domain and a short intracellular chain (not shown here). As shown here, the [alpha]-3 domain is capable of binding to the receptors ILT2 (see Shiroishi et al., Proc Natl Acad Sci U S A. 2003 July 22;100(15):8856-8861) and ILT4 (see Shiroishi et al., Proc Natl Acad Sci U S A. 2006 Oct 31;103(44):16412-7) on immune cells. Physiologically, these sequences form a non-covalently linked MHC class 1 complex. To simplify purification of the complex MHC Ib molecule, one or more protein tags (such as SpotTag, myc tag and/or His(6x) tag) may be introduced. They may be introduced in such a way as to enable their later optional removal via cleavage using an optional Factor Xa cleavage site. Furthermore, the antigenic peptide, beta 2-microglobulin and MHC Ib [alpha]chain can be linked in order to increase the stability. The vector map was generated using Snapgene Viewer Software.

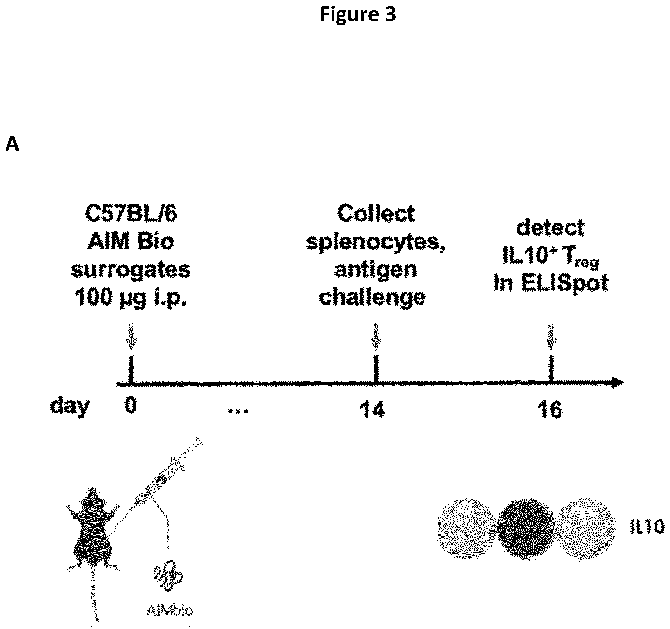

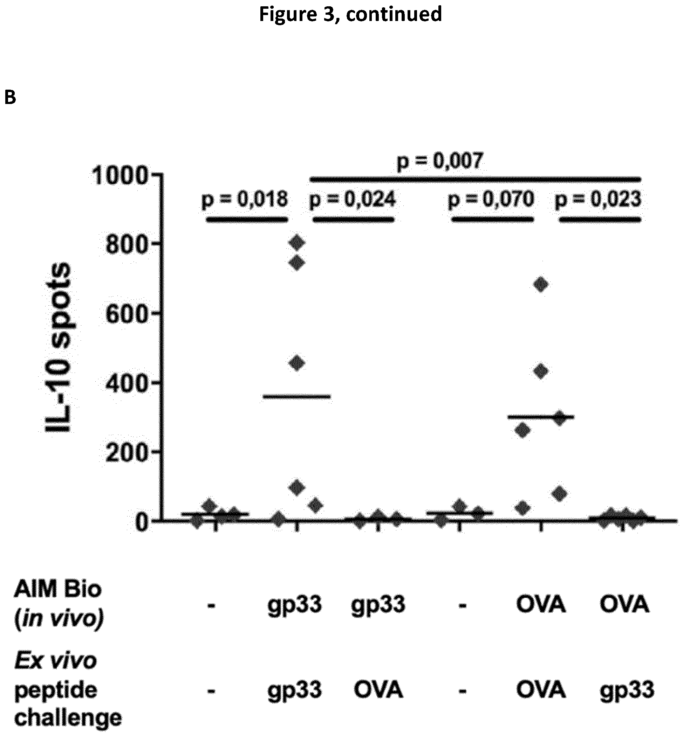

Figure 3: Surrogates of recombinant polypeptides of the invention induce IL10 secreting Treg in mice.
In this experiment, 100µg of surrogate molecules consisting of a viral (Gp33) or Ovalbumin (Ova) model peptide antigen, murine H2-K^{b} alpha1 and 2 domains, and human HLA-G alpha 3 domain and beta-2-microglobulin were injected i.p. into 12 week old C57BL/6 mice. After 14 days, mice were sacrificed and splenocytes were rechallenged with 5µg/ml of either Gp33 or Ova peptide in an 48h standard murine IL-10 ELIspot assay (Mabtech mouse IL-10 HRP ELISpot kit, cells cultured in RPMI10% FCS 10ng/ml IL2). A significant increase in regulatory T cells that secreted IL-10 only in response to rechallenge with the peptide towards which tolerance was induced via surrogate molecule injection was detectable.
(A) experimental design; (B) results
Figure 4: Surrogates of recombinant polypeptides of the invention prevent CD8+T-cell driven EAE in mice. In this EAE mouse model, the model antigen ovalbumin (OVA) is expressed in oligodendrocytes under the control of the myelin basic protein (MBP) promoter (ODC-OVA). This leads to the presentation of the OVA257-264 peptide on H-2Kb MHC molecules on oligodendrocytes. OT-I mice express a T cell receptor (OT-I) on their CD8+ T cells, which recognizes exactly this peptide-MHC combination. When CD8+ T cells from these mice are transferred into 10 day old ODC-OVA mice, these develop an experimental autoimmune encephalomyelitis (EAE) which resembles in many aspects the pathogenesis and symptomatology of MS (Na et al., Brain, Volume 131, Issue 9, September 2008, Pages 2353-2365). In this experiment, 500µg of surrogate molecules consisting of a viral (Gp33) or Ovalbumin (Ova) model peptide antigen, murine H2-K^{b} alpha1 and 2 domains, and human HLA-G alpha 3 domain and beta-2-microglobulin or just PBS were injected the same day. EAE was scored according to Bittner et al., J Vis Exp . 2014 Apr 15;(86):51275.
Only Ovalbumin-tolerance inducing surrogate molecules almost completely prevented EAE symptoms. (A) experimental design; (B) results
Figure 5: Some surrogates of recombinant polypeptides of the invention selectively prevent CD4+ T cell driven EAE in mice.
In this model, a strong, myelin-specific autoimmune response is triggered by administration of MOG 35-55 peptide in combination with Complete Freund's adjuvant, which activates CD4+ Th17 cells, and pertussis toxin, which makes the blood-brain barrier more permeable (Protocol: Bittner et al., J Vis Exp . 2014 Apr 15;(86):51275). Here, CD4+ cells as well as antibodies play a crucial role in the development of EAE (Tigno-Aranjuez et al., J Immunol November 1, 2009, 183 (9) 5654-5661). In addition, 100µg/mouse of surrogate molecules consisting of a viral (Gp33) or two Mog peptide antigens (Mog37 or Mog44), murine H2-D^{b} alpha1 and 2 domains, and human HLA-G alpha 3 domain and beta-2-microglobulin or just PBS were injected the first day.
The Mog44 peptide containing surrogate molecule significantly reduced EAE symptoms and weight loss. (A) experimental design; (B) EAE score; (C) body weight
Figure 6: Mog44 surrogates of recombinant polypeptides of the invention prevented inflammation and CD8 T cell infiltration in the spinal cord.
10 µm fresh frozen sections were stained with comercial Toluidine 1x staining reagent for 1h at room temperature. A strong infiltration of immune cells was detected in EAE, but prevented by Mog44_Db_G. 10 µm fresh frozen sections were briefly dried at room temperature, fixed with acetone, blocked with 5% BSA 10% normal goat serum in PBS, stained with 1:100 anti-CD8 antibody, secondary antibody coupled to HRP and DAB solution (detailed methods: Karikari et al., Brain Behav Immun. 2022 Jan 12;101:194-210). Mog35-55 induced EAE lead to a strong infiltration of CD8+ cells into the spinal cord which was prevented by MOG44_Db_G surrogate molecule.
(A) Toluidine; (B) CD8-DAB
Figure 7: Detection of anti-MOG35-55 antibodies in Mog-EAE mice treated with surrogates of recombinant polypeptides of the invention ("AIM Bio")
Murine serum was collected from heart puncture after mice were sacrificed. 10µg/ml Mog35-55 were used for coating over night, wells were blocked using 1%BSA, and anti-Mog35-55 antibodies were detected using the indicated secondary HRP coupled antibodies.
Mog35-55 induced EAE correlated with high levels of Mog35-55 specific IgG autoantibodies, which which were not detectable in animals treated with 100µg MOG44_Db_G surrogate molecule.

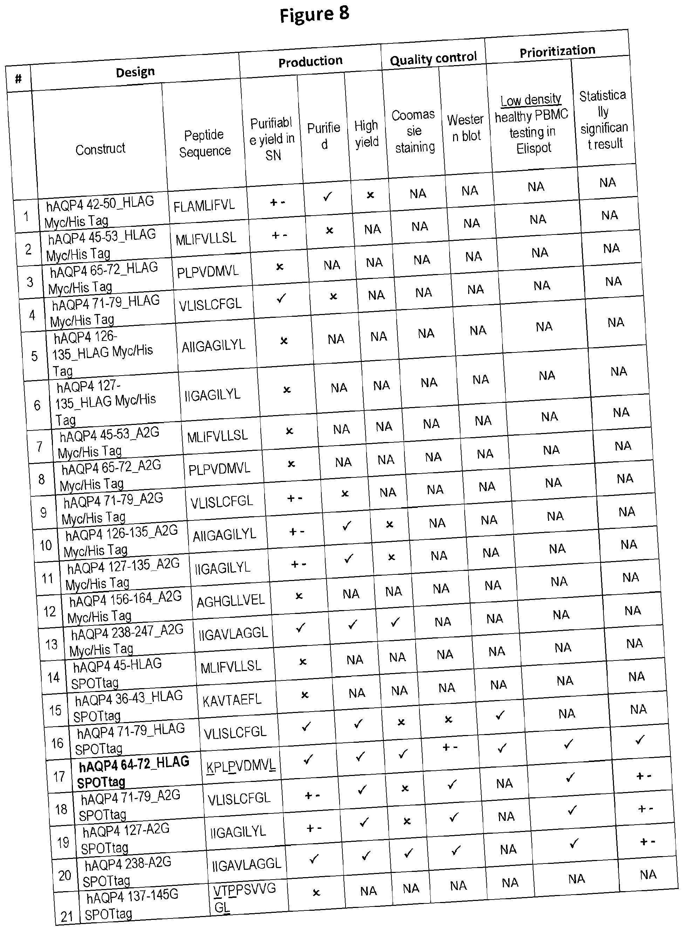

Figure 8: List of the human NMO recombinant polypeptide candidates.
The peptide antigen sequences of the NMO recombinant polypeptide candidates shown in the Figure are as follows:

| Construct | Peptide antigen | SEQ ID NO: |
|---|---|---|
| hAQP4 42-50_HLAG Myc/His Tag | FLAMLIFVL | SEQ ID NO: 31 |
| hAQP4 45-53_HLAG Myc/His Tag | MLIFVLLSL | SEQ ID NO: 32 |
| hAQP4 65-72_HLAG Myc/His Tag | PLPVDMVL | SEQ ID NO: 33 |
| hAQP4 71-79_HLAG Myc/His Tag | VLISLCFGL | SEQ ID NO: 22 |
| hAQP4 126-135_HLAG Myc/His Tag | AIIGAGILYL | SEQ ID NO: 34 |
| hAQP4 127-135_HLAG Myc/His Tag | IIGAGILYL | SEQ ID NO: 23 |
| hAQP4 45-53_A2G Myc/His Tag | MLIFVLLSL | SEQ ID NO: 32 |
| hAQP4 65-72_A2G Myc/His Tag | PLPVDMVL | SEQ ID NO: 33 |
| hAQP4 71-79_A2G Myc/His Tag | VLISLCFGL | SEQ ID NO: 22 |
| hAQP4 126-135_A2G Myc/His Tag | AIIGAGILYL | SEQ ID NO: 34 |
| hAQP4 127-135_A2G Myc/His Tag | IIGAGILYL | SEQ ID NO: 23 |
| hAQP4 156-164_A2G Myc/His Tag | AGHGLLVEL | SEQ ID NO: 35 |
| hAQP4 238-247_A2G Myc/His Tag | IIGAVLAGGL | SEQ ID NO: 24 |
| hAQP4 45-HLAG SPOTtag | MLIFVLLSL | SEQ ID NO: 32 |
| hAQP4 36-43_HLAG SPOTtag | KAVTAEFL | SEQ ID NO: 36 |
| hAQP4 71-79_HLAG SPOTtag | VLISLCFGL | SEQ ID NO: 22 |
| hAQP4 64-72_HLAG SPOTtag | KPLPVDMVL | SEQ ID NO: 2 |
| hAQP4 71-79_A2G SPOTtag | VLISLCFGL | SEQ ID NO: 22 |
| hAQP4 127-A2G SPOTtag | IIGAGILYL | SEQ ID NO: 23 |
| hAQP4 238-A2G SPOTtag | IIGAVLAGGL | SEQ ID NO: 24 |
| hAQP4 137-145_HLAG SPOTtag | VTPPSWGGL | SEQ ID NO: 37 |

Figure 8 further shows which combinations of AQP4 peptides and antigen-presenting MHC class I alpha 1 and 2 domains (HLAg= HLA-G, A2G = HLA-A2 presenting domains + HLA-G alpha 3 domain) lead to successful (✔ ) intermediate (+ -) or non-preferable (×) results with regards to production, quality control and prioritization in healthy blood donors (described in Figure 9)
Figure 9: Upregulation of CD8 Treg in healthy blood donors by a recombinant polypeptide of the invention containing the KPLPVDMVL antigen ("AQP_64")
In vitro Treg induction mediated by peptide-HLA-G containing constructs (AIM Biologicals) was carried out as follows: PBMCs from healthy donors were purified via density centrifugation performed on white blood cells from a leukocyte reduction chamber using Ficoll. Cells were centrifuged for 20 min at 1200 x g without brake followed by collection of the interphase ring that was washed with 1x PBS (5 min, 300 x g). PBMC were frozen till further use.
PBMCs were thawed 1 day prior to PBMC pulsing (d-1) and kept over night in 5 ml X-VIVO 15 medium containing 5% human AB serum in a well of a 6 well plate at 37°C.
On the next day (d0), cells were counted and resuspended in X-VIVO 15 complete medium (5% hAB serum & cytokine cocktail: 20 ng/ml hIL-2, 20 ng/ml hGM-CSF, 10 ng/ml hIL-4 & 10 ng/ml hTGF-b1) at a cell density of 3×10⁶ cells/ml. For experiments, 3×10⁶ cells were seeded in the respective wells of a 12-well plate with a final volume of 1000 µl X-VIVO complete medium with cytokine cocktail and 5 µg/ml of an AIM Bio molecule or the respective controls.
On day 3, 1 ml complete medium (with cytokines) was added, on day 6, a second pulse with 5 µg/ml AIM Bio molecule was performed (after removing medium). On days 7, 10 & 12, 1 ml complete medium (with cytokines) was added.
On day 13, ELISpot plate PVDF membrane was activated with 50 µl/well EtOH (35% v/v) for 1 min followed by 5x washing with 200 µl distilled sterile water. Plate was coated with 100 µl/well anti-hIL10 (clone 9D-7, 1:500 dilution in PBS, sterile filtered) at 4°C over night. On the next day, unbound coating antibody was removed, 5 washing steps were performed with 200 µl PBS and 200 µl blocking buffer (X-VIVO 15 5% hAB serum) was added and the plate incubated for 30 min - 2 h at room temperature.
Day 14, 200,000 cells were seeded per well on the ELISpot plates in duplicates, including negative controls (cells plus PBS) and a positive control (e.g. LPS) for 48h.
Secondary antibody was prepared: 1 µg/ml aIL-10-biotinylated antibody in 0.5% BSA/1x PBS (1:1000 dilution) and horseradish peroxidase-conjugated streptavidin (1:750 in 0.5% BSA/PBS), tetramethylbenzidine solution was filtered using a 0.45 µm filter and stored at 4°C till use.
Cell supernatant was removed and 5x washed using 100 µl PBS. Last excess buffer was removed using paper towels. 25 µl diluted HRP-streptavidin (1:750) was added per well and incubated for 1 h at room temperature in the dark followed by 5 washing steps using sterile 1xPBS.
100 µl of filtered TMB substrate was added per well for 15-25 min till blue sports developed. Reaction was stopped by washing the wells thoroughly with water.
Plastic underdrains of the plates were removed and the bottom and sides of the plates were also washed with tap water and dried.
AQP4_64_G_Spt induced at least 30% more IL-10 secreting T reg in 65% of of all healthy blood donors.

### DETAILED DESCRIPTION OF THE INVENTION

### Definitions and General Techniques

Unless otherwise defined below, the terms used in the present invention shall be understood in accordance with their common meaning known to the person skilled in the art. All publications, patents and patent applications cited herein are hereby incorporated by reference in their entirety for all purposes. Publications referred to herein may be cited by specifying the full literature reference in the text.

All proteins in accordance with the invention, including the recombinant polypeptides of the invention, can be obtained by methods known in the art. Such methods include methods for the production of recombinant polypeptides. The recombinant polypeptides of the invention can be expressed in recombinant host cells according to the invention. Recombinant host cells of the invention are preferably mammalian cells such as CHO and HEK cells.

It will be understood that the recombinant polypeptides of the invention are meant to optionally include a secretion signal peptide sequence. Similarly, the recombinant polypeptides of the invention are meant to also optionally include affinity tags, e.g. in order to facilitate purification, and optional protease cleavage sites between the tag and the polypeptide, e.g. in order to facilitate removal of the tags by protease cleavage. It is also understood that any reference to amino acid sequences referred to herein is meant to encompass not only the unmodified amino acid sequence but also typical posttranslational modifications of these amino acid sequences (e.g., glycosylation or deamidation of amino acids, the clipping of particular amino acids or other posttranslational modifications) occurring in cellular expression systems known in the art, including mammalian cells such as CHO and HEK cells.

Likewise, it will be understood that the recombinant polypeptides of the invention are meant to optionally include the respective pro-peptides.

It will also be understood that the recombinant polypeptides of the invention can be in form of their soluble or their membrane-bound form. Whether a recombinant polypeptide is "soluble" under these conditions can be determined by methods known in the art, e.g., by measuring the turbidity of the recombinant polypeptide under the above-indicated reference conditions. As used herein, soluble means that at least 95% of the recombinant polypeptide is determined to be soluble under these reference conditions.

Single chain MHC molecules can be stored, for instance, in PBS at -80°C (with or without 0.1% human albumin as carrier, depending on the protein concentration) or in 50% glycerol at -20°C.

According to the invention, MHC molecules are preferably human MHC molecules.

The recombinant polypeptides of the invention are preferably isolated recombinant polypeptides.

It will be understood how a recombinant polypeptide capable of binding and presenting an peptide antigen according to the invention can be prepared. For example, peptide antigen-binding domains such as [alpha]1 and [alpha]2 domains are well-known, and modifications of these domains can be made. The capability of a peptide antigen to bind to the polypeptides and MHC molecules according to the invention can be determined by techniques known in the art, including but not limited to explorative methods such as MHC peptide elution followed by Mass spectrometry and bio-informatic prediction in silico, and confirmative methods such as MHC peptide multimere binding methods and stimulation assays.

It will be understood that in connection with the peptide antigens used in accordance with the invention, any lenghts of these peptide antigens referred to herein (e.g. "7 to 11 amino acids in length") are meant to refer to the length of the peptide antigens themselves. Thus, the lenghts of peptide antigens referred to herein do not include the length conferred by additional amino acids which are not part of the peptide antigens such as additional amino acids from possible linker sequences etc.

In accordance with the present invention, each occurrence of the term "comprising" may optionally be substituted with the term "consisting of".

### Methods and Techniques

Generally, unless otherwise defined herein, the methods used in the present invention (e.g. cloning methods or methods relating to antibodies) are performed in accordance with procedures known in the art, e.g. the procedures described in Sambrook et al. ("Molecular Cloning: A Laboratory Manual.", 2nd Ed., Cold Spring Harbor Laboratory Press, Cold Spring Harbor, New York 1989), Ausubel et al. ("Current Protocols in Molecular Biology." Greene Publishing Associates and Wiley Interscience; New York 1992), and Harlow and Lane ("Antibodies: A Laboratory Manual" Cold Spring Harbor Laboratory Press, Cold Spring Harbor, New York 1988), all of which are incorporated herein by reference.

Protein-protein binding, such as binding of antibodies to their respective target proteins, can be assessed by methods known in the art. Protein-protein binding is preferably assessed by surface plasmon resonance spectroscopy measurements.

For instance, binding of MHC class Ib molecules or recombinant polypeptides according to the invention to their receptors, including ILT2 and ILT4, is preferably assessed by surface plasmon resonance spectroscopy measurements. More preferably, binding of MHC class Ib molecules or recombinant polypeptides according to the invention to their receptors is assessed by surface plasmon resonance measurements at 25°C. Appropriate conditions for such surface plasmon resonance measurements have been described by Shiroishi et al., Proc Natl Acad Sci U S A. 2003 July 22;100(15):8856-8861.

Sequence Alignments of sequences according to the invention are performed by using the BLAST algorithm (see Altschul et al.(1990) "Basic local alignment search tool." Journal of Molecular Biology 215. p. 403-410.; Altschul et al.: (1997) Gapped BLAST and PSI-BLAST: a new generation of protein database search programs. Nucleic Acids Res. 25:3389-3402.). Appropriate parameters for sequence alignments of short peptides by the BLAST algorithm, which are suitable for peptide antigens in accordance with the invention, are known in the art. Most software tools using the BLAST algorithm automatically adjust the parameters for sequence alignments for a short input sequence. In one embodiment, the following parameters are used: Max target sequences 10; Word size 3; BLOSUM 62 matrix; gap costs: existence 11, extension 1; conditional compositional score matrix adjustment. Thus, when used in connection with sequences, terms such as "identity" or "identical" preferably refer to the identity value obtained by using the BLAST algorithm.

### Preparation of pharmaceutical compositions of the Invention

Pharmaceutical compositions of the present invention are prepared in accordance with known standards for the preparation of pharmaceutical compositions.

For instance, the pharmaceutical compositions are prepared in a way that they can be stored and administered appropriately. The pharmaceutical compositions of the invention may therefore comprise pharmaceutically acceptable components such as carriers, excipients and/or stabilizers.

Such pharmaceutically acceptable components are not toxic in the amounts used when administering the pharmaceutical composition to a human patient. The pharmaceutical acceptable components added to the pharmaceutical compositions may depend on the chemical nature of the active ingredients present in the composition, the particular intended use of the pharmaceutical compositions and the route of administration. In general, the pharmaceutically acceptable components used in connection with the present invention are used in accordance with knowledge available in the art, e.g. from Remington's Pharmaceutical Sciences, Ed. AR Gennaro, 20th edition, 2000, Williams & Wilkins, PA, USA. Pharmaceutical compositions comprising the nucleic acids of the invention (e.g., RNAs) may also be formulated in accordance with knowledge available in the art, e.g. using liposomal formulations targeting dendritic cells.

### Peptide Antigens in Accordance with the Invention

The peptide antigens which can be used in accordance with the invention, including the peptide antigens as defined above, are not particularly limited other than by their ability to be presented on MHC molecules. It is understood that a "peptide antigen presented by said recombinant polypeptide" as referred to in relation to the invention is a peptide antigen that is presented by said recombinant polypeptide to human T cells, if such T cells are present, in a way that it binds to a T cell receptor on the human T-cells.

Peptides which are able to be presented on MHC molecules can be generated as known in the art (see, for instance, Rammensee, Bachmann, Emmerich, Bachor, Stevanovic. SYFPEITHI: database for MHC ligands and peptide motifs. Immunogenetics. 1999 Nov;50(3-4):213-9; Pearson et al. MHC class I-associated peptides derive from selective regions of the human genome. J Clin Invest. 2016 Dec 1;126(12):4690-4701; and Rock, Reits, Neefjes. Present Yourself! By MHC Class I and MHC Class II Molecules. Trends Immunol. 2016 Nov;37(11):724-737).

Peptide antigens are generally known in the art. Generally, the peptide antigens in accordance with the invention are capable of binding to MHC class I proteins. It will be understood by a person skilled in the art that for each MHC class Ib molecule or polypeptide capable of presenting peptides in accordance with the invention, peptide antigens which are capable of binding to said MHC class Ib molecule or recombinant polypeptide will preferably be used. These peptide antigens can be selected based on methods known in the art.

Binding of peptide antigens to MHC class Ib molecules or to polypeptides capable of peptide antigen binding in accordance with the invention can be assessed by methods known in the art, e.g. the methods of:
Rammensee, Bachmann, Emmerich, Bachor, Stevanovic. SYFPEITHI: database for MHC ligands and peptide motifs. Immunogenetics. 1999 Nov;50(3-4):213-9;
Pearson et al. MHC class I-associated peptides derive from selective regions of the human genome. J Clin Invest. 2016 Dec 1;126(12):4690-4701; and
Rock, Reits, Neefjes. Present Yourself! By MHC Class I and MHC Class II Molecules. Trends Immunol. 2016 Nov;37(11):724-737.

Such methods include experimental methods and methods for the prediction of peptide antigen binding. Anchor residues which serve to anchor the peptide antigen on the MHC class I molecule and to ensure binding of the peptide antigen to the MHC class I molecule are known in the art.

In a preferred embodiment in accordance with all embodiments of the invention, the peptide antigen used in accordance with the invention contain any of the anchor or preferred amino acid residues in the positions as predicted for MHC class I molecules.

Such predictions can preferably be made in as described in any one of the following publications:
- Rammensee et al, SYFPEITHI: database for MHC ligands and peptide motifs. Immunogenetics (1999) 50: 213-219
- Nielsen et al, Protein Sci (2003) 12:1007-1017
- Neefjes et al. Nat Rev Immunol. 2011 Nov 11;11(12):823-36
- Diehl et al. Curr Biol. 1996 Mar 1;6(3):305-14,
- Lee et al. Immunity. 1995 Nov;3(5):591-600.
- Desai & Kulkarni-Kale, T-cell epitope prediction methods: an overview. Methods Mol Biol. 2014;1184:333-64.
- Jumper et al. Highly accurate protein structure prediction with AlphaFold. Nature 2021;596:583-589

In the invention, the peptide antigen is from human aquaporin 4.

It is understood that the non-anchor amino acid residues of the peptide antigen of the invention may or may not contain conservative substitutions, preferably not more than two conservative substitutions, more preferably one conservative subsitution with respect to the corresponding amino acid sequence of a peptide antigen from human aquaporin 4.

Peptide antigens of the invention preferably consist of naturally occurring amino acids. However, non-naturally occurring amino acids such as modified amino acids can also be used. For instance, in one embodiment, a peptide antigen of the invention encompasses the peptidomimetic of the indicated peptide antigen amino acid sequence of human aquaporin 4.

Methods for the synthesis of peptide antigens, including peptide antigens in accordance with the invention, are well known in the art.

### Therapeutic applications of the invention

The recombinant polypeptides of the invention can be used for the treatment of neuromyelitis optica.

The treatment can be a treatment by inducing myelin-specific regulatory T cells. As such regulatory T cells (e.g., CD8-positive regulatory T cells) are activated in myelinated structures, they confer target cell protection against cytotoxic T cells recognizing the same or another myelin antigen. Regulatury T-cells (e.g., CD8-positive regulatory T cells) are known in the art and can be detected, for instance, by their secretion of IL-10.

While CD8-positive regulatory T cells are not as well known as CD4CD25 regulatory T cells, they have even been described to be more potent. See, for instance:
Junfeng Liu , Dacan Chen, Golay D. Nie and Zhenhua Dai CD8+CD122+ T-Cells: A Newly Emerging Regulator with Central Memory Cell Phenotypes. Front. Immunol. doi: 10.3389/fimmu.2015.00494; and
Niederlova, V., Tsyklauri, O., Chadimova, T. and Stepanek, O. (2021), CD8+ Tregs revisited: A heterogeneous population with different phenotypes and properties. Eur. J. Immunol., 51: 512-530. https://doi.org/10.1002/eji.202048614

While these are characterized by expression of CD122 and CD8 in mice, their human counterparts have been described to be CD8 and CXCR3 positive. See, for instance:
Shi Z, Okuno Y, Rifa'i M, Endharti AT, Akane K, Isobe K, et al. Human CD8+CXCR3+ T cells have the same function as murine CD8+CD122+Treg. Eur J Immunol (2009) 39:2106-2119. doi: 10.1002/eji.200939314).
The treatment according to the invention can be a treatment for reducing plasma or cerebrospinal fluid (CSF) levels of autoantibodies against human aquaporin 4. The human patient can be a patient who had plasma or cerebrospinal fluid (CSF) autoantibodies against human aquaporin 4 prior to the start of the treatment.

In accordance with the invention, the autoantibodies can be detected by various methods known in the art. A preferred approach are cell-based assays (CBAs) where the suspected target antigen of the autoantibodies (e.g., aquaporin 4) is overexpressed in HEK293 or CHO cells which are then incubated with serum or cerebrospinal fluid, typically for 1 h at room temperature. Mock-transfected sister cells serve as controls. Autoantibodies that bind to the cells are detected with different fluorescently labeled anti-human specific secondary antibodies that recognize total human IgG (heavy and light chain), IgG-Fc (constant chain) or IgG1. Binding is quantified by either flow cytometry (CBA-FACS) or visual scoring by microscopic evaluation of the immunofluorescence (CBA-IF), which is often titrated. Other approaches like enzyme-linked immunosorbent assays (ELISAs) or Western Blots are also possible, but often less sensitive, as conformation-sensitive antibodies may not be detected by these methods. Suitable approaches have been described in

Waters, P., Pettingill, P. & Lang, B. Detection methods for neural autoantibodies. Handb. Clin. Neurol.133, 147-163 (2016).

Methods for the detection of Aqp4-specific autoantibodies are described in
Lennon, V. A., Kryzer, T. J., Pittock, S. J., Verkman, A. S. & Hinson, S. R. IgG marker of optic-spinal multiple sclerosis binds to the aquaporin-4 water channel. J. Exp. Med.202, 473-477 (2005).

### Sequences

Preferred amino acid sequences referred to in the present application can be independently selected from the following sequences. The sequences are represented in an N-terminal to C-terminal order; and they are represented in the one-letter amino acid code.

Exemplary sequences which are part of of the recombinant polypeptides of the invention:
Optional leader Peptide (absent from the recombinant polypeptide due to processing during cellular expression): e.g. MSRSVALAVLALLSLSGLEA (SEQ ID NO: 1)
Peptide antigen: any MHC class I peptide corresponding to MHC class I [alpha] 1&2 domains, e.g. KPLPVDMVL (SEQ ID NO: 2)
First linker: For instance GGGGSGGGGSGGGGS (SEQ ID NO: 3) or GCGASGGGGSGGGGS (SEQ ID NO: 4)
beta 2 Microglobulin, for instance: 

 (SEQ ID NO: 5, human beta 2 Microglobulin)
Second Linker, for instance:
   GGGGSGGGGSGGGGSGGGGS (SEQ ID NO: 6)
[Alpha] 1 & 2 domain derived either from human HLA-G or from any other MHC class I [alpha]1&2 domain suitable to present the selected antigenic peptide, Y84 may be C in DT variant or A
e.g. [Alpha] 1 & 2 domain derived from human HLA-G: E.g.
   GSHSMRYFSAAVSRPGRGEPRFIAMGYVDDTQFVRFDSDSACPRMEPRAPWVEQEGPEYWEEETRNTKAH AQTDRMNLQTLRGCYNQSEASSHTLQWMIGCDLGSDGRLLRGYEQYAYDGKDYLALNEDLRSWTAADTAA QISKRKCEAANVAEQRRAYLEGTCVEWLHRYLENGKEMLQRA (SEQ ID NO: 7)
Or: Human HLA-A2 [alpha]1 & 2 domain: E.g.
   GSHSMRYFFTSVSRPGRGEPRFIAVGYVDDTQFVRFDSDAASQRMEPRAPWIEQEGPEYWDGETRKVKAH SQTHRVDLGTLRGCYNQSEAGSHTVQRMYGCDVGSDWRFLRGYHQYAYDGKDYIALKEDLRSWTAADMAA QTTKHKWEAAHVAEQLRAYLEGTCVEWLRRYLENGKETLQRT (SEQ ID NO: 8)
Human HLA-G [alpha]3 domain (or any MHC Ib [alpha]3 domain, such as HLA-F, which also interacts with ILT2 and ILT4 receptors), for instance:
   DPPKTHVTHHPVFDYEATLRCWALGFYPAEIILTWQRDGEDQTQDVELVETRPAGDGTFQKWAAVWPSGE EQRYTCHVQHEGLPEPLMLRWSKEGDGGIMSVRESRSLSEDL (SEQ ID NO: 9; sequence of HLA-G [alpha]3).

Note that the following underlined amino acids of this sequence are relevant for ILT2 or ILT4 receptor interaction:

DPPKTHVTHHPVFDYEATLRCWALGFYPAEIILTWQRDGEDQTQDVELVETRPAGDGTFQKWAAVVVPSGE
EQRYTCHVQHEGLPEPLMLRWSKEGDGGIMSVRESRSLSEDL

Alternatively, a shorter form of a human HLA-G [alpha]3 domain may be used which lacks the optional C-terminal amino acid sequence from intron 4 (SKEGDGGIMSVRESRSLSEDL; SEQ ID NO: 20), i.e.: 
DPPKTHVTHHPVFDYEATLRCWALGFYPAEIILTWQRDGEDQTQDVELVETRPAGDGTFQKWAAVVVPSGE
EQRYTCHVQHEGLPEPLMLRW (SEQ ID NO: 21),
Factor Xa restriction site: IEGRTGTKLGP (SEQ ID NO: 10)
SpotTag: PDRVRAVSHWSSC (SEQ ID NO: 11)
Myc tag: EQKLISEEDL (SEQ ID NO: 12)
His tag: HHHHHH* (SEQ ID NO: 13)
Spacer sequence: e.g. NSAVD (SEQ ID NO: 14) or GS

Further (alternative) exemplary peptide antigens which can be part of the recombinant polypeptides of the invention are as follows:
VLISLCFGL (SEQ ID NO: 22), preferably in recombinant polypeptides containing HLA-G alpha1 and 2 domains
IIGAGILYL (SEQ ID NO: 23), preferably in recombinant polypeptides containing HLA-A2 alpha1 and 2 domains
IIGAVLAGGL (SEQ ID NO: 24), preferably in recombinant polypeptides containing HLA-A2 alpha1 and 2 domains

Example for a recombinant polypeptide of the invention (with the optional leader peptide):

MSRSVALAVLALLSLSGLEAKPLPVDMVLGCGASGGGGSGGGGSIQRTPKIQVYSRHPAENGKSNFLNCYVS
GFHPSDIEVDLLKNGERIEKVEHSDLSFSKDWSFYLLYYTEFTPTEKDEYACRVNHVTLSQPKIVKWDRDMGG
GGSGGGGSGGGGSGGGGSGSHSMRYFSAAVSRPGRGEPRFIAMGYVDDTQFVRFDSDSACPRMEPRAP
WVEQEGPEYWEEETRNTKAHAQTDRMNLQTLRGCYNQSEASSHTLQWMIGCDLGSDGRLLRGYEQYAYD
GKDYLALNEDLRSWTAADTAAQISKRKCEAANVAEQRRAYLEGTCVEWLHRYLENGKEMLQRADPPKTHVT
HHPVFDYEATLRCWALGFYPAEIILTWQRDGEDQTQDVELVETRPAGDGTFQKWAAVVVPSGEEQRYTCHV
QHEGLPEPLMLRWSKEGDGGIMSVRESRSLSEDLGSPDRVRAVSHWSSC* (SEQ ID NO: 15; note that the
asterisk denotes the stop codon)

Note that the sequence of the peptide antigen (here: KPLPVDMVL) of the above full length recombinant polypeptide can be substituted by any peptide antigen sequence in accordance with the invention, i.e. by any peptide antigen presented by said recombinant polypeptide, wherein the peptide antigen is a peptide of human aquaporin 4. That is, recombinant polypeptides of the invention may consist of a sequence consisting of a peptide antigen which is a peptide of human aquaporin 4 (e.g., any one of the peptide antigens of SEQ ID NOs: 2, 22, 23, and 24), followed by the sequence of

GCGASGGGGSGGGGSIQRTPKIQVYSRHPAENGKSNFLNCYVSGFHPSDIEVDLLKNGERIEKVEHSDLSFS
KDWSFYLLYYTEFTPTEKDEYACRVNHVTLSQPKIVKWDRDMGGGGSGGGGSGGGGSGGGGSGSHSMRY
FSAAVSRPGRGEPRFIAMGYVDDTQFVRFDSDSACPRMEPRAPWVEQEGPEYWEEETRNTKAHAQTDRMN
LQTLRGCYNQSEASSHTLQWMIGCDLGSDGRLLRGYEQYAYDGKDYLALNEDLRSWTAADTAAQISKRKCE
AANVAEQRRAYLEGTCVEWLHRYLENGKEMLQRADPPKTHVTHHPVFDYEATLRCWALGFYPAEIILTWQRD
GEDQTQDVELVETRPAGDGTFQKWAAVVVPSGEEQRYTCHVQHEGLPEPLMLRWSKEGDGGIMSVRESRS
LSEDLGSPDRVRAVSHWSSC* (SEQ ID NO: 16; note that the asterisk denotes the stop codon)

These recombinant polypeptides of the invention may also contain the optional leader peptide as exemplified above.

The receptors ILT2 (also known as LILRB1) and ILT4 (also known as LILRB2) are known in the art. Preferred sequences of these receptors in accordance with the invention are as follows:
ILT2: 
MTPILTVLICLGLSLGPRTHVQAGHLPKPTLWAEPGSVITQGSPVTLRCQGGQETQEYRL
YREKKTALWITRIPQELVKKGQFPIPSITWEHAGRYRCYYGSDTAGRSESSDPLELVVTG
AYIKPTLSAQPSPVVNSGGNVILQCDSQVAFDGFSLCKEGEDEHPQCLNSQPHARGSSRA
IFSVGPVSPSRRWWYRCYAYDSNSPYEWSLPSDLLELLVLGVSKKPSLSVQPGPIVAPEE
TLTLQCGSDAGYNRFVLYKDGERDFLQLAGAQPQAGLSQANFTLGPVSRSYGGQYRCYGA
HNLSSEWSAPSDPLDILIAGQFYDRVSLSVQPGPTVASGENVTLLCQSQGWMQTFLLTKE
GAADDPWRLRSTYQSQKYQAEFPMGPVTSAHAGTYRCYGSQSSKPYLLTHPSDPLELVVS
GPSGGPSSPTTGPTSTSGPEDQPLTPTGSDPQSGLGRHLGVVIGILVAVILLLLLLLLLF
LILRHRRQGKHWTSTQRKADFQHPAGAVGPEPTDRGLQWRSSPAADAQEENLYAAVKHTQ
PEDGVEMDTRSPHDEDPQAVTYAEVKHSRPRREMASPPSPLSGEFLDTKDRQAEEDRQMD
TEAAASEAPQDVTYAQLHSLTLRREATEPPPSQEGPSPAVPSIYATLAIH (SEQ ID NO: 17)
ILT4:

MTPIVTVLICLGLSLGPRTHVQTGTIPKPTLWAEPDSVITQGSPVTLSCQGSLEAQEYRL
YREKKSASWITRIRPELVKNGQFHIPSITWEHTGRYGCQYYSRARWSELSDPLVLVMTGA
YPKPTLSAQPSPVVTSGGRVTLQCESQVAFGGFILCKEGEEEHPQCLNSQPHARGSSRAI
FSVGPVSPNRRWSHRCYGYDLNSPYVWSSPSDLLELLVPGVSKKPSLSVQPGPVVAPGES
LTLQCVSDVGYDRFVLYKEGERDLRQLPGRQPQAGLSQANFTLGPVSRSYGGQYRCYGAH
NLSSECSAPSDPLDILITGQIRGTPFISVQPGPTVASGENVTLLCQSWRQFHTFLLTKAG
AADAPLRLRSIHEYPKYQAEFPMSPVTSAHAGTYRCYGSLNSDPYLLSHPSEPLELVVSG
PSMGSSPPPTGPISTPAGPEDQPLTPTGSDPQSGLGRHLGVVIGILVAVVLLLLLLLLLF

LILRHRRQGKHWTSTQRKADFQHPAGAVGPEPTDRGLQWRSSPAADAQEENLYAAVKDTQ
PEDGVEMDTRAAASEAPQDVTYAQLHSLTLRRKATEPPPSQEREPPAEPSIYATLAIH (SEQ ID NO: 18)

The sequence of human aquaporin 4 is known in the art. Preferred amino acid sequences of human aquaporin 4 are as follows:
>sp|P55087-2|AQP4_HUMAN Isoform 1 of Aquaporin-4 OS=Homo sapiens OX=9606 GN=AQP4 
MVAFKGVWTQAFWKAVTAEFLAMLIFVLLSLGSTINWGGTEKPLPVDMVLISLCFGLSIA
TMVQCFGHISGGHINPAVTVAMVCTRKISIAKSVFYIAAQCLGAIIGAGILYLVTPPSVV
GGLGVTMVHGNLTAGHGLLVELIITFQLVFTIFASCDSKRTDVTGSIALAIGFSVAIGHL
FAINYTGASMNPARSFGPAVIMGNWENHWIYWVGPIIGAVLAGGLYEYVFCPDVEFKRRF
KEAFSKAAQQTKGSYMEVEDNRSQVETDDLILKPGVVHVIDVDRGEEKKGKDQSGEVLSS
V
(SEQ ID NO: 19)
>sp|P55087|AQP4_HUMAN Aquaporin-4 Isoform 2 OS=Homo sapiens OX=9606 GN=AQP4 PE=1 SV=2 
MSDRPTARRWGKCGPLCTRENIMVAFKGVWTQAFWKAVTAEFLAMLIFVLLSLGSTINWG
GTEKPLPVDMVLISLCFGLSIATMVQCFGHISGGHINPAVTVAMVCTRKISIAKSVFYIA
AQCLGAIIGAGILYLVTPPSVVGGLGVTMVHGNLTAGHGLLVELIITFQLVFTIFASCDS
KRTDVTGSIALAIGFSVAIGHLFAINYTGASMNPARSFGPAVIMGNWENHWIYWVGPIIG
AVLAGGLYEYVFCPDVEFKRRFKEAFSKAAQQTKGSYMEVEDNRSQVETDDLILKPGVVH
VIDVDRGEEKKGKDQSGEVLSSV
(SEQ ID NO: 29)
>NP_001304313.1 aquaporin-4 isoform M1x [Homo sapiens]

MSDRPTARRWGKCGPLCTRENIMVAFKGVWTQAFWKAVTAEFLAMLIFVLLSLGSTINWGGTEKPLPVDM
VLISLCFGLSIATMVQCFGHISGGHINPAVTVAMVCTRKISIAKSVFYIAAQCLGAIIGAGILYLVTPPS
VVGGLGVTMVHGNLTAGHGLLVELIITFQLVFTIFASCDSKRTDVTGSIALAIGFSVAIGHLFAINYTGA
SMNPARSFGPAVIMGNWENHWIYWVGPIIGAVLAGGLYEYVFCPDVEFKRRFKEAFSKAAQQTKGSYMEV
EDNRSQVETDDLILKPGVVHVIDVDRGEEKKGKDQSGEVLSSVXLEDRTESRQDSLELSSDFLPPIKETD
LL
(SEQ ID NO: 30; where X can be tryptophan, cysteine, arginine or serine or a stop codon)

The present invention is further illustrated by the following non-limiting examples:

### EXAMPLES

### Example 1:

### Methods for producing recombinant polypeptides of the invention

- Expi-293F cells (Thermo Fisher), grown in Expi-293^{™} expression medium (Thermo Fisher): transfection of 1 µg DNA into 2.5×10⁶ cells/ml using the Expifectamine^{™} 293 Transfection kit (Thermo Fisher) using Opti-MEM (Thermo Fisher) for complexation of DNA with Expifectamine, after 18-20 h, addition of enhancer according to the protocol, harvesting of the supernatant after 4-6 days (37°C, 8% CO₂, humidified incubator), 19 mm² orbital shaker 125 rpm
- Spot-tag protein purification: equilibration of Spot-Cap resin: transfer of desired slurry amount into an appropriate tube, sediment beads by centrifugation (4°C, 4 min, 2500 g), remove & discard supernatant, add 10 bed volumes PBS (cold) to beads, invert to mix, sediment beads by centrifugation (4°C, 4 min, 2500 g), remove & discard supernatant, repeat 2 times
- Add required volume beads to supernatant, incubate ON, 4°C on a rotator, wash beads by repeated centrifugation (4°C, 4 min, 2500 g), and removal of supernatant
- Prepare a 500 µM Spot-peptide solution in PBS, remove the supernatant, incubate with 1/3rd of the spot-peptide solution for 5-10 min

Sediment beads by centrifugation. Use Amicon Ultra-4 centrifugal filters (15 kDa cutoff) for Protein concentration and spot-peptide removal with 15 kDa Amicon cutoff columns

Rinse the Amicon Ultra-4 centrifugal filters (15 kDa cutoff) with PBS followed by 0.1 N NaOH (centrifugation at 4000 g, 4°C) to remove trace amounts of glycerine.

### ELISPOT:

### 1) Cell culture

### A) PBMC isolation (under a laminar flow hood)

To isolate peripheral blood mononuclear cells (PBMC), a density centrifugation was performed with white blood cells from a leukocyte reduction chamber and density gradient medium (e.g. Ficoll, or ROTI Sep 1077). Cells were centrifuged for 20 min at 1200 x g without brake followed by collection of the interphase ring that was washed with 1x PBS (5 min, 300 x g). PBMC were frozen till further use.

### B) PBMC pulsing (under a laminar flow hood)

PBMCs were thawed 1 day prior to PBMC pulsing (d-1) and kept over night in 5 ml X-VIVO 15 medium containing 5% human AB serum in a well of a 6 well plate at 37°C.

On the next day (d0) cells were counted and resuspended in X-VIVO 15 complete medium (5% hAB serum & cytokine cocktail: 20 ng/ml hIL-2, 20 ng/ml hGM-CSF, 10 ng/ml hIL-4 & 10 ng/ml hTGF-b1) at a cell density of 3×10⁶ cells/ml.

For experiments, 3×10⁶ cells were seeded in the respective wells of a 12-well plate with a final volume of 1000 µl X-VIVO complete medium with cytokine cocktail and
5 µg/ml of an AIM Bio molecule or the respective controls.

On day 3, 1 ml complete medium (with cytokines) was added, on day 6, a second pulse with 5 µg/ml of a recombinant polypeptide of the invention or a surrogate thereof (collectively referred to as "AIM Bio" molecule) was performed (after removing medium). On days 7, 10 & 12, 1 ml complete medium (with cytokines) was added.

### Required:

X-VIVO 15 medium + 5% human AB serum
X-VIVO 15 complete medium: X-VIVO 15 medium + 2% human AB serum supplemented with cytokine cocktail: 10 ng/ml TGF-b1, 10 ng/ml IL-4, 20 ng/ml IL-2, 20 ng/ml GM-CSF

### 2) ELISPOT

### Laminar flow hood

On day 13, ELISPOT plates were coated using anti-hIL10 (clone 9D-7, 1:500 dilution in PBS, sterile filtered) and aIL10 (10G8-biotin) and on day 14, 200,000 cells were seeded per well on the ELISPOT plates in duplicates, including negative controls (cells plus PBS) and a positive control (e.g. LPS).

The PFDF membrane was activated with 50 µl/well EtOH (35% v/v) for 1 min followed by 5x washing with 200 µl distilled sterile water. Plate was coated with 100 µl/well antibody solution at 4°C over night. On the next day, unbound coating antibody was removed, 5 washing steps were performed with 200 µl PBS and 200 µl blocking buffer (X-VIVO 15 5% hAB serum) was added and the plate incubated for 30 min - 2 h at room temperature.

The respective antigenic peptide in DMSO or DMSO as a control were prepared, and a final amount of 5 µg peptide/ml was added to the final volume of 100 µl/well. 150,000 cells were seeded per well in X-VIVO 15 medium with 5% human AB serum. Blocking buffer (X VIVO 15 medum + 5% hAB serum) was carefully removed, and medium with PBS as negative control and stimulants (5 µg/ml total volume in each well) were added to the other wells and incubated at 37°C over night.

### Outside the laminar flow hood

Secondary antibody was prepared: 1 µg/ml aIL-10-biotinylated antibody in 0.5% BSA/1x PBS (1:1000 dilution) and horseradish peroxidase-conjugated streptavidin (1:750 in 0.5% BSA/PBS), tetramethylbenzidine solution was filtered using a 0.45 µm filter and stored at 4°C till use.

Cell supernatant was removed and 5x washed using 100 µl PBS. Last excess buffer was removed using paper towels.
25 µl diluted HRP-streptavidin (1:750) was added per well and incubated for 1 h at room temperature in the dark followed by 5 washing steps using sterile 1xPBS.
100 µl of filtered TMB substrate was added per well for 15-25 min till blue sports developed. Reaction was stopped by washing the wells thoroughly with tapped water.

Plastic underdrains of the plates was removed and the bottom and sides of the plates were washed with tap water and dried.

Plates were read out using an ImmunoSpot S6 Ultra-V Analyzer (Cellular Technology Limited), analysed in Excel and graphs/statistics were done in Graphad Prism.

### Required:

Capture antibodies: anti-hIL10 (Clone: 9D-7, Mabtech #3430-3-250; 1:500 dilution), anti-hIL10-biotinylated (Mabtech, #3430-6-250)
1x PBS (sterile)
35% EtOH (v/v)
Blocking buffer: X-vivo 5% hAB serum (sterile) [blocking is done in the same medium as cell culture]
Dilution buffer: 0.5% BAS in PBS
Washing buffer: 1x PBS
Medium: for T cells, X-VIVO 15 medium (Lonza)
Filter syringe: Millex GV
ELISPOT PVDF plate (#MSIP4510, Millipore)
TMB substrate

### Example 2: Surrogates of recombinant polypeptides of the invention induce IL10 secreting Treg in mice

Wild type black 6 mice were injected with 100µg recombinant polypeptides (also referred to as "AIMBio") having the following sequences,
Ova_KbG 
SIINFEKLGCGASGGGGSGGGGSIQRTPKIQVYSRHPAENGKSNFLNCYVSGFHPSDIEVDLLKNGERIEKVE
HSDLSFSKDWSFYLLYYTEFTPTEKDEYACRVNHVTLSQPKIVKWDRDMGGGGSGGGGSGGGGSGGGGS
GPHSLRYFVTAVSRPGLGEPRYMEVGYVDDTEFVRFDSDAENPRYEPRARWMEQEGPEYWERETQKAKG
NEQSFRVDLRTLLGCYNQSKGGSHTIQVISGCEVGSDGRLLRGYQQYAYDGCDYIALNEDLKTWTAADMAAL
ITKHKWEQAGEAERLRAYLEGTCVEWLRRYLKNGNATLLRTDPPKTHVTHHPVFDYEATLRCWALGFYPAEII
LTWQRDGEDQTQDVELVETRPAGDGTFQKWAAVVVPSGEEQRYTCHVQHEGLPEPLMLRWSKEGDGGIM
SVRESRSLSEDLGSPDRVRAVSHWSSC (SEQ ID NO: 25),
 and
Gp33_KbG 
AVYNFATMGCGASGGGGSGGGGSIQRTPKIQVYSRHPAENGKSNFLNCYVSGFHPSDIEVDLLKNGERIEKV
EHSDLSFSKDWSFYLLYYTEFTPTEKDEYACRVNHVTLSQPKIVKWDRDMGGGGSGGGGSGGGGSGGGGS
GPHSLRYFVTAVSRPGLGEPRYMEVGYVDDTEFVRFDSDAENPRYEPRARWMEQEGPEYWERETQKAKG
NEQSFRVDLRTLLGCYNQSKGGSHTIQVISGCEVGSDGRLLRGYQQYAYDGCDYIALNEDLKTWTAADMAAL
ITKHKWEQAGEAERLRAYLEGTCVEWLRRYLKNGNATLLRTDPPKTHVTHHPVFDYEATLRCWALGFYPAEII
LTWQRDGEDQTQDVELVETRPAGDGTFQKWAAVVVPSGEEQRYTCHVQHEGLPEPLMLRWSKEGDGGIM
SVRESRSLSEDL GSPDRVRAVSHWSSC (SEQ ID NO: 26),
 for inducing tolerance towards an OVA peptide or an viral gp33 peptide, respectively. After 2 weeks, mice were sacrificed, and splenocytes re-challenged either with the matching or a mismatching peptide. IL-10 secreting cells were quantified by ELIspot. The results are shown in Figure 3.

### Example 3: Surrogates of recombinant polypeptides of the invention selectively prevent CD8+ T-cell driven EAE in mice

As described in (Na et al, Brain. 2008 Sep;131 (Pt 9):2353-65.), the adoptive transfer of CD8+ OT-I T cells that recognize an ovalbumin epitope in the context of H2-K^{b} into mice which express ovalbumin in oligodendrocytes leads to experimental autoimmune encephalomyelitis which recapitulates many MS and late stage NMO symptoms. In this animal model, a single injection of 500µg of recombinant polypeptides surrogate molecules (also referred to as "AIMBio") that induce tolerance towards the targeted ovalbumin epitope almost completely prevented EAE symptoms, while a surrogate molecule presenting a control peptide hat no significant protective effects (Figure 4). The sequences of the recombinant polypeptide surrogate molecules are listed in Example 2.

### Example 4: Some surrogates of recombinant polypeptides of the invention selectively prevent CD4+ T cell driven EAE in mice

At the day of the 33µg or 100µg recombinant polypeptide of the invention surrogate molecule ("AIM Bio") i.p. injection, 100 µl MOG35-55 peptide/CFA (Complete Freund's Adjuvance; final concentration Mycobacterium Tuberculosis H37RA and peptide each 1 mg/ml) emulsion were injected each left and right s.c. into the flank and 250ng pertussis toxin (in 200µl PBS) intraperitoneally. A second pertussis toxin injection was given 3 days later. In this animal model, a single injection AIM Bio surrogate molecules that induce tolerance towards the a Mog epitope (Mog44_Kb_G) significantly reduced EAE symptoms, while a surrogate molecule presenting a control peptide (Gp33) or a non-functional Mog peptide (Mog37) hat no significant protective effects (Figure 5). In this model Mog44 AIM Bio also prevented inflammation and CD8 T cell infiltration in the spinal cord (Figure 6). The sequences of the recombinant polypeptide surrogate molecules were listed in example 2 or as follows:
Mog44_DbG 
FSRVVHLYRNGGCGASGGGGSGGGGSIQRTPKIQVYSRHPAENGKSNFLNCYVSGFHPSDIEVDLLKNGERI
EKVEHSDLSFSKDWSFYLLYYTEFTPTEKDEYACRVNHVTLSQPKIVKWDRDMGGGGSGGGGSGGGGSGG
GGSGPHSMRYFETAVSRPGLEEPRYISVGYVDNKEFVRFDSDAENPRYEPRAPWMEQEGPEYWERETQKA
KGQEQWFRVSLRNLLGCYNQSAGGSHTLQQMSGCDLGSDWRLLRGYLQFAYEGRDYIALNEDLKTWTAAD
MAAQITRRKWEQSGAAEHYKAYLEGECVEWLHRYLKNGNATLLRTDPPKTHVTHHPVFDYEATLRCWALGF
YPAEIILTWQRDGEDQTQDVELVETRPAGDGTFQKWAAVVVPSGEEQRYTCHVQHEGLPEPLMLRWSKEGD
GGIMSVRESRSLSEDLGSPDRVRAVSHWSSC (SEQ ID NO: 27)
Mog37_DbG

VGWYRSPFSRGCGASGGGGSGGGGSIQRTPKIQVYSRHPAENGKSNFLNCYVSGFHPSDIEVDLLKNGERI
EKVEHSDLSFSKDWSFYLLYYTEFTPTEKDEYACRVNHVTLSQPKIVKWDRDMGGGGSGGGGSGGGGSGG
GGSGPHSMRYFETAVSRPGLEEPRYISVGYVDNKEFVRFDSDAENPRYEPRAPWMEQEGPEYWERETQKA
KGQEQWFRVSLRNLLGCYNQSAGGSHTLQQMSGCDLGSDWRLLRGYLQFAYEGRDYIALNEDLKTWTAAD
MAAQITRRKWEQSGAAEHYKAYLEGECVEWLHRYLKNGNATLLRTDPPKTHVTHHPVFDYEATLRCWALGF
YPAEIILTWQRDGEDQTQDVELVETRPAGDGTFQKWAAVVVPSGEEQRYTCHVQHEGLPEPLMLRWSKEGD
GGIMSVRESRSLSEDLGSPDRVRAVSHWSSC (SEQ ID NO: 28)

In this model Mog44 AIM Bio also completely prevented the formation of MOG-specific autoantibodies in the serum as tested by ELISA (Figure 7). This is a strong indicator that the recombinant polypeptides of the invention are effective therapeutics in NMO, which are often characterized by antibody responses against human aquaporin 4. Thus, the patient population is defined by a common autoimmune-related antigen. Certain MHC molecules are also associated with NMO.

Mog-reactive antibodies in sera of AIM Bio (33 or 100µg) treated mice were detected via standard ELISA protocol, with 3 washes in between each step. Briefly, ELISA plates were coated with 10µg/ml Mog35-55 peptide, blocked with PBS 1% BSA, before mouse sera diluted 1:25 in PBS 1% BSA were added for 1h. Anti-mouse IgG-HRP or anti-mouse heavy and light chain - HRP antibodies diluted 1:5000 were used for detection.

### Example 5: Human recombinant polypeptide candidates of the invention for NMO

The recombinant polypeptides of the invention are newly developed protein complexes derived from the pregnancy-associated immunosuppressive MHC molecule HLA-G. It is likely that HLA-G enables an embryo to influence the maternal immune system to tolerate embryonic antigens but further antagonize antigens from pathogens. The recombinant polypeptides of the invention containing variable peptides were able to selectively eliminate peptide-specific cytotoxic effector T cells as well as induce peptide-specific regulatory T cells in the test tube.

Figure 8 shows a list of the human MS & MOGAD recombinant polypeptide candidates.

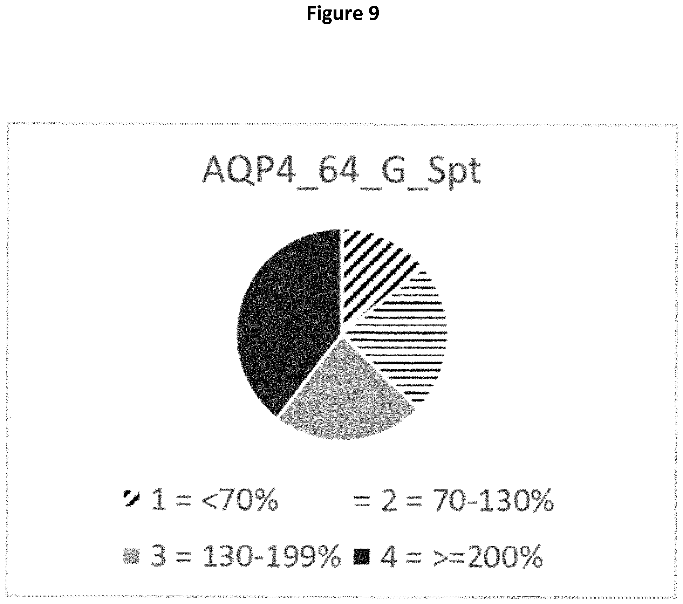

The inventors' findings show that single-chain proteins containing AQP4 peptide antigens and a HLA-G alpha 3 domain can induce tolerogenic T cells in healthy donors. Thus, CD8 Treg were upregulated by at least 30% in 65% of of all healthy blood donors (Figure 9).

### INDUSTRIAL APPLICABILITY

The pharmaceutical compositions, polypeptides, nucleic acids, cells, and products for use in the invention are industrially applicable. For example, they can be used in the manufacture of, or as, pharmaceutical products.

## Claims

1. A recombinant polypeptide capable of presenting a peptide antigen, the recombinant polypeptide comprising, in an N- to C-terminal order,
i) a peptide antigen presented by said recombinant polypeptide, wherein the peptide antigen is a peptide of human aquaporin 4;
ii) optionally a linker sequence;
iii) optionally a sequence of a human polypeptide domain comprising a sequence of a human β2 microglobulin, or an amino acid sequence at least 90% identical to the amino acid sequence of human β2 microglobulin represented by SEQ ID NO: 5;
iv) optionally a linker sequence;
v) optionally an [alpha] 1 domain of an MHC molecule;
vi) optionally an [alpha] 2 domain of an MHC molecule;
vii) an [alpha] 3 domain of an MHC class Ib molecule or a derivative of an [alpha] 3 domain of an MHC class Ib molecule, said derivative being capable of binding to ILT2 or ILT4;
viii) optionally a protease cleavage site;
ix) optionally a spacer sequence; and
x) optionally an affinity tag.

2. The recombinant polypeptide according to claim 1, wherein said peptide antigen according to i) is 7 to 11 amino acids in length, preferably 8-10 amino acids in length.

3. The recombinant polypeptide according to claim 1 or 2, wherein said peptide antigen according to i) consists of an amino acid sequence selected from the group consisting of the amino acid sequences of SEQ ID NOs: 2, 22, 23 and 24.

4. The recombinant polypeptide according to any one of the preceding claims, wherein said [alpha]1 domain according to (v) and said [alpha]2 domain according to (vi) are from a human MHC class la molecule, preferably a human HLA-A2 molecule, or from a human MHC class Ib molecule, preferably a human HLA-G molecule.

5. The recombinant polypeptide according to any one of the preceding claims, wherein the [alpha] 3 domain of the MHC class Ib molecule according to (vii) is an [alpha] 3 domain of human HLA-E, human HLA-F or human HLA-G, preferably human HLA-G.

6. The recombinant polypeptide according to any one of the preceding claims, wherein the [alpha]3 domain or derivative according to (vii) is identical to or has at least 80% amino acid sequence identity, at least 90% amino acid sequence identity, at least 92% amino acid sequence identity, at least 94% amino acid sequence identity, at least 96% amino acid sequence identity, at least 98% amino acid sequence identity, or at least 99% amino acid sequence identity with the [alpha]3 domain having the amino acid sequence of SEQ ID NO: 9 or SEQ ID NO: 21 or is identical to the [alpha]3 domain having the amino acid sequence of SEQ ID NO: 9 or SEQ ID NO: 21.

7. The recombinant polypeptide according to any one of the preceding claims, wherein the linker sequence according to (ii) and/or the linker sequence according to (iv) comprises the amino acid sequence (GGGGS)n, wherein n is an integer equal to or higher than 1, and wherein n is an integer selected from the group consisting of 1, 2, 3, 4, 5, 6, 7, 8, 9 and 10 and is preferably selected from the group consisting of 2, 3, 4 and 5.

8. The recombinant polypeptide according to any one of the preceding claims, wherein said sequence of a human polypeptide domain according to (iii) is at least 95% identical to the amino acid sequence of SEQ ID NO: 5, preferably at least 98% identical to the amino acid sequence of SEQ ID NO: 5 and more preferably identical to the amino acid sequence of SEQ ID NO: 5.

9. The recombinant polypeptide according to any one of the preceding claims, wherein said polypeptide is dimeric or multimeric.

10. The recombinant polypeptide according to any one of the preceding claims, wherein the polypeptide comprises or consists of all of the components i) to vii), wherein the polypeptide does not comprise components viii) to x), or wherein the polypeptide comprises or consists of all of the components i) to x).

11. The recombinant polypeptide according to any one of the preceding claims, further comprising an N-terminal secretion signal peptide sequence.

12. The recombinant polypeptide according to any one of claims 1-10, wherein the recombinant polypeptide consists of an amino acid sequence consisting of the following ((a) and (b)) in an N-to C-terminal order:
(a) a peptide antigen selected from the group consisting of the amino acid sequences of SEQ ID NOs: 2, 22, 23 and 24, and
(b) the amino acid sequence of SEQ ID NO: 16.

13. The recombinant polypeptide according to any one of the preceding claims, wherein the recombinant polypeptide is soluble.

14. A nucleic acid encoding one or more polypeptides according to any one of the preceding claims, wherein the nucleic acid is preferably a vector.

15. A pharmaceutical composition or kit comprising at least one nucleic acid according to claim 14.

16. A pharmaceutical composition or kit comprising at least one recombinant polypeptide according to any one of claims 1-13.

17. The pharmaceutical composition or kit according to claim 16, wherein the pharmaceutical composition or kit comprises at least two different recombinant polypeptides according to any one of claims 1-13, and wherein each of the different polypeptides comprises a different peptide antigen as defined in claim 3.

18. A pharmaceutical composition or kit according to any one of claims 15-17, for use in the treatment of neuromyelitis optica in a human patient.

19. The pharmaceutical composition or kit for use according to claim 18, wherein the treatment is treatment by immunotherapy, and wherein the treatment is preferably by inducing immunological tolerance against human aquaporin 4.

20. The pharmaceutical composition or kit for use according to any one of claims 18-19, wherein the treatment is for reducing plasma or cerebrospinal fluid levels of autoantibodies against human aquaporin 4, and wherein the human patient is a patient who had plasma or cerebrospinal fluid autoantibodies against human aquaporin 4 prior to the start of the treatment.

21. The pharmaceutical composition or kit for use according to any one of claims 18-20, wherein the treatment is by inducing myelin-specific regulatory T cells.

22. A recombinant host cell comprising a nucleic acid or a vector according to claim 14 and expressing the recombinant polypeptide according to any one of claims 1-13.

23. A method for obtaining pharmaceutical composition comprising a polypeptide according to any one of claims 1-13, the method comprising the steps of (a) culturing the recombinant host cell of claim 22 under conditions allowing expression of the recombinant polypeptide from the nucleic acid molecule, (b) recovering the recombinant polypeptide, (c) purifying the recombinant polypeptide, and (d) formulating the recombinant polypeptide into a pharmaceutical composition.
